# EUROPEAN PATENT APPLICATION

(11) **EP 4 273 246 A1**
(43) Date of publication of application: **08.11.2023**
(21) Application number: 21914494.6
(22) Date of filing: 29.12.2021
(51) Int. Cl.: C12N 15/31, C07K 14/34, C12N 15/77, C12P 13/18, C12R 1/13, C12N 1/21, C12R 1/15

(54) **RECOMBINANT STRAIN OF MODIFYING GENE BBD29_14900, AND CONSTRUCTION METHOD AND USE THEREOF**

(30) Priority: 30.12.2020 CN 202011628757
(71) Applicant: Ningxia Eppen Biotech Co. Ltd, Ningxia 750100 (CN)
(72) Inventor: MA, Fengyong, Yinchuan, Ningxia 750100 (CN); WEI, Aiying, Yinchuan, Ningxia 750100 (CN); MENG, Gang, Yinchuan, Ningxia 750100 (CN); ZHAO, Chunguang, Yinchuan, Ningxia 750100 (CN); JIA, Huiping, Yinchuan, Ningxia 750100 (CN); SU, Houbo, Yinchuan, Ningxia 750100 (CN); YANG, Lipeng, Yinchuan, Ningxia 750100 (CN); GUO, Xiaowei, Yinchuan, Ningxia 750100 (CN); TIAN, Bin, Yinchuan, Ningxia 750100 (CN); ZHOU, Xiaoqun, Yinchuan, Ningxia 750100 (CN)
(74) Representative: ABG Intellectual Property Law, S.L.
(86) International application number: PCT/CN2021/142439
(87) International publication number: WO 2022/143762

(57) **Abstract**

Provided are a recombinant strain with modified gene BBD29_14900, and a method for constructing the same and use thereof, with the production of L-glutamic acid as a specific application. Further provided is a method for introducing a point mutation into the BBD29_14900 gene coding sequence in *Corynebacterium* or improving the expression thereof. The method can cause a bacterial strain with the mutation to increase the fermentation yield of glutamic acid. The point mutation involves a mutation of the base at position 1114 in the sequence of the BBD29_14900 gene from guanine (G) to adenine (A), and thus a substitution of aspartic acid at position 372 in the coded corresponding amino acid sequence with asparagine.

## Description

### Technical Field

The present invention belongs to the technical field of gene engineering and microorganisms, and in particular relates to recombinant strain with modified gene BBD29_14900, and method for constructing the same and use thereof, with the production of L-glutamic acid as a specific application.

### Background Art

As is well known, MSG, a daily seasoning, is a monosodium salt of L-glutamic acid. Since the invention and industrialized production of MSG in 1909 in Japan, it has developed and evolved into a worldwide amino acid fermentation industry with the fermentation of glutamic acid as a theme. Fermentation of MSG-producing amino acids such as glutamic acid, distinguished from conventional fermentation for brewing and antibiotics, is a fermentation with controlled metabolism in which microbial metabolism is changed.

Currently, the strains used by manufacturers for glucose fermentation are primarily some mutant ones, and the strains for production are basically obtained from microbial breeding with mutagenesis as a main approach. It has been quite difficult, however, in continued employment of classic microbial breeding techniques such as mutagenesis, cell fusion, etc. for intended substantial increases in the rate of amino acid production and the rate of glucose-to-amino acid conversion given the current capacity in the amino acid production. Therefore, it is the most effective approach currently to improve the rate of amino acid production and the rate of glucose-to-amino acid conversion with modified strains by taking advantages of modern gene engineering.

If gene engineering as a measure can be taken to modify strains for production, a great breakthrough will be made in the rate of glutamic acid production and the rate of glucose-to-glutamic acid conversion.

### Summary

The present invention provides recombinant strain with modified gene BBD29_14900, and method for constructing the same and use thereof.

The objective of the present invention is to develop a new technique for enhancing L-glutamic acid production capacity of a bacterium, thereby providing a method for producing L-glutamic acid effectively.

In order to achieve the above objective, the inventors of the present invention have found in research that the gene BBD29_14900 or a homologous gene thereof can be modified, or improved with respect to the expression thereof to provide a bacterium having enhanced capacity in L-glutamic acid production. The present invention is completed in view of these discoveries.

The present invention provides a bacterium for generating L-glutamic acid, wherein the expression of a polynucleotide encoding an amino acid sequence of SEQ ID NO: 3 or a homologous sequence thereof is improved. The present invention further provides a method for producing L-glutamic acid with the microorganism.

A first aspect of the present invention provides a bacterium for generating L-glutamic acid, having an improved expression of a polynucleotide encoding an amino acid sequence of SEQ ID NO: 3 or a homologous sequence thereof. According to the present invention, the improved expression is an enhanced expression of the polynucleotide, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

The amino acid sequence of SEQ ID NO: 3 or the homologous sequence thereof is a protein coded by the gene BBD29_14900 or a homologous gene thereof.

The bacterium has an enhanced L-glutamic acid production capacity compared with an unmodified strain.

In the present invention, the term "bacterium having L-glutamic acid production capacity" refers to a bacterium having such a capacity of producing and accumulating target L-glutamic acid in a culture medium and/or in a cell of the bacterium that L-glutamic acid can be collected when the bacterium is cultured in the culture medium. A bacterium having L-glutamic acid production capacity can be a bacterium capable of accumulating target L-glutamic acid in a culture medium and/or in a cell of the bacterium in an amount greater than that with an unmodified strain.

The term "unmodified strain" refers to a control strain which has not been modified in a manner such that a specific feature is incorporated. That is, examples of the unmodified strain comprise a wild-type strain and a parent strain.

A bacterium having L-glutamic acid production capacity can be a bacterium capable of accumulating target L-glutamic acid in a culture medium in an amount, preferably above 0.5 g/L, and more preferably above 1.0 g/L.

In the present invention, the term "L-glutamic acid" refers to L-glutamic acid in a free form, a salt thereof or a mixture thereof, unless otherwise specified.

The polynucleotide can encode an amino acid sequence having about 90% or more, about 92% or more, about 95% or more, about 97% or more, about 98% or more, or about 99% or more sequence homology with an amino acid sequence of SEQ ID NO: 3. As used herein, the term "homology" refers to percentage identity between two polynucleotides or two polypeptides as modules. Sequence homology can be measured between one module and another module using a method known in the art. For example, such sequence homology can be measured by virtue of BLAST algorithm.

The expression of a polynucleotide can be enhanced: by substituting or mutating an expression regulatory sequence, introducing a mutation to the sequence of the polynucleotide, and by increasing the copy number of the polynucleotide inserted via a chromosome or introduced with a vector, or a combination thereof, etc.

The expression regulatory sequence of a polynucleotide can be modified. The expression regulatory sequence controls the expression of a polynucleotide operably linked thereto, and can comprise, for example, promoters, terminators, enhancers, silencers and the like. A polynucleotide can have a change in an initiation codon. A polynucleotide can be incorporated into a specific site of a chromosome, thereby increasing copy number. Herein, a specific site can comprise, for example, a transposon site or an intergenic site. In addition, a polynucleotide can be incorporated into an expression vector, and the expression vector is introduced into a host cell, thus increasing copy number.

In an embodiment of the present invention, a polynucleotide, or a polynucleotide having a point mutation is incorporated into a specific site of a chromosome of a microorganism, thus increasing copy number.

In an embodiment of the present invention, a polynucleotide with a promoter sequence, or a polynucleotide having a point mutation with a promoter sequence is incorporated into a specific site of a chromosome of a microorganism, thus overexpressing the nucleic sequence.

In an embodiment of the present invention, a polynucleotide, or a polynucleotide having a point mutation is incorporated into an expression vector, and the expression vector is introduced into a host cell, thus increasing copy number.

In an embodiment of the present invention, a polynucleotide with a promoter sequence, or a polynucleotide having a point mutation with a promoter sequence is incorporated into an expression vector, and the expression vector is introduced into a host cell, thus overexpressing the amino acid sequence.

In a specific embodiment of the present invention, the polynucleotide prior to mutation can comprise a polynucleotide sequence of SEQ ID NO: 1.

In an embodiment of the present invention, the polynucleotide encoding an amino acid sequence of SEQ ID NO: 3 has a point mutation such that aspartic acid (D) at position 372 in the amino acid sequence of SEQ ID NO: 3 is substituted with a different amino acid.

According to the present invention, preferably aspartic acid at position 372 is substituted with asparagine.

According to the present invention, as set forth in SEQ ID NO: 4 is the amino acid sequence following substitution with asparagine of aspartic acid at position 372 in an amino acid sequence set forth in SEQ ID NO: 3.

In an embodiment of the present invention, the polynucleotide sequence having a point mutation is formed from a mutation to the base at position 1114 of a polynucleotide sequence set forth in SEQ ID NO: 1.

According to the present invention, the mutation comprises a mutation of the base at position 1114 of a polynucleotide sequence set forth in SEQ ID NO: 1 from guanine (G) to adenine (A).

In an embodiment of the present invention, the polynucleotide sequence having a point mutation comprises a polynucleotide sequence set forth in SEQ ID NO: 2.

As used herein, the term "operably linked" refers to a functional link between a regulatory sequence and a polynucleotide sequence, whereby the regulatory sequence controls transcription and/or translation of the polynucleotide sequence. A regulatory sequence can be a strong promoter which can enhance the expression level of a polynucleotide. A regulatory sequence can be a promoter derived from a microorganism belonging to the genus *Corynebacterium* or can be a promoter derived from other microorganisms. For example, the promoter can be trc promoter, gap promoter, tac promoter, T7 promoter, lac promoter, trp promoter, araBAD promoter or cj7 promoter.

In a specific embodiment of the present invention, the promoter is a promoter of a polynucleotide encoding an amino acid sequence of SEQ ID NO: 3 (the gene BBD29_14900).

As used herein, the term "vector" refers to a polynucleotide construct containing a regulatory sequence of a gene and a gene sequence and configured to express a target gene in a suitable host cell. Or, a vector can also refer to a polynucleotide construct containing a sequence for homologous recombination such that due to the vector introduced into a host cell, the regulatory sequence of an endogenous gene in the genome of the host cell can be changed, or a target gene that can be expressed can be inserted into a specific site of the genome of a host. In this regard, the vector used in the present invention can further comprise a selective marker to determine the introduction of the vector into a host cell or the insertion of the vector into the chromosome of a host cell. A selective marker can comprise a marker providing a selectable phenotype, such as drug resistance, auxotroph, resistance to cytotoxic agents, or expression of a surface protein. In an environment treated with such a selective agent, the transformed cell can be selected since only the cell that expresses the selective marker can survive or display different phenotypic traits.

In some specific embodiments of the present invention, the vector used is pK18mobsacB plasmid, and pXMJ19 plasmid.

As used herein, the term "transformation" refers to introduction of a polynucleotide into a host cell, such that the polynucleotide can be replication-competent as an element foreign to a genome or by being inserted into the genome of a host cell. A method for transforming the vector used in the present invention can comprise a method for introducing a nucleic acid into a cell. In addition, as disclosed in the related techniques, a method with electric pulse can be performed according to a host cell.

According to the present invention, the bacterium can be a microorganism belonging to the genus *Corynebacterium,* such as *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis,* etc.

In an embodiment of the present invention, the microorganism belonging to the genus *Corynebacterium* is *Corynebacterium glutamicum* ATCC13869.

In an embodiment of the present invention, the microorganism belonging to the genus *Corynebacterium* is *Corynebacterium glutamicum* YPGLU001, which yields high production of glutamic acid, and was deposited with Strain Name: *Corynebacterium glutamicum;* Latin Name: *Corynebacterium glutamicum;* Strain No.: YPGLU001 in China General Microbiological Culture Collection Center, abbreviated as CGMCC, Address: No. 3, Yard 1, Beichen West Road, Chaoyang District, Beijing on November 23, 2020, with Accession No.: CGMCC No. 21220.

According to the present invention, the bacterium can also have additional improvements relating to the increase in the production of L-glutamic acid, such as enhancement or reduction in the activities of, or in the expressions of the genes of glutamate dehydrogenase, glutamine synthetase, glutamine-oxoglutaric acid amino transferase, etc., or substitution of the genes with foreign genes.

A second aspect of the present invention provides a polynucleotide sequence, an amino acid sequence coded by the polynucleotide sequence, a recombinant vector comprising the polynucleotide sequence, and a recombinant strain containing the polynucleotide sequence.

According to the present invention, the polynucleotide sequence comprises a polynucleotide encoding a polypeptide containing an amino acid sequence set forth in SEQ ID NO: 3 with aspartic acid at position 372 in the sequence substituted with a different amino acid.

According to the present invention, preferably aspartic acid at position 372 is substituted with asparagine.

According to the present invention, as set forth in SEQ ID NO: 4 is the amino acid sequence following substitution with asparagine of aspartic acid at position 372 in an amino acid sequence set forth in SEQ ID NO: 3.

According to the present invention, preferably the polynucleotide encoding a polypeptide containing an amino acid sequence set forth in SEQ ID NO: 3 contains a polynucleotide sequence as set forth in SEQ ID NO: 1.

In an embodiment of the present invention, the polynucleotide sequence is formed from a mutation to the base at position 1114 in a polynucleotide sequence set forth in SEQ ID NO: 1.

According to the present invention, the mutation refers to a change in the base/nucleotide of the site, and the method for mutation can be at least one selected from mutagenesis, PCR site-directed mutation, and/or homologous recombination, etc. In the present invention, PCR site-directed mutation and/or homologous recombination are preferably used.

According to the present invention, the mutation comprises a mutation of the base at position 1114 of a polynucleotide sequence set forth in SEQ ID NO: 1 from guanine (G) to adenine (A).

In an embodiment of the present invention, the polynucleotide sequence comprises a polynucleotide sequence set forth in SEQ ID NO: 2.

According to the present invention, the amino acid sequence comprises an amino acid sequence set forth in SEQ ID NO: 4.

According to the present invention, the recombinant vector is constructed by introducing the polynucleotide sequence to a plasmid.

In an embodiment of the present invention, the plasmid is pK18mobsacB plasmid.

In another embodiment of the present invention, the plasmid is pXMJ19 plasmid.

Specifically, the polynucleotide sequence and the plasmid can be constructed as a recombinant vector by virtue of NEBuider recombination system.

According to the present invention, the recombinant strain contains the polynucleotide sequence.

As an embodiment of the present invention, a starting strain of the recombinant strain is *Corynebacterium glutamicum* CGMCC No. 21220.

As an embodiment of the present invention, a starting strain of the recombinant strain is ATCC 13869.

A third aspect of the present invention further provides a method for constructing a recombinant strain generating L-glutamic acid.

According to the present invention, the method for constructing comprises the following step of:
engineering a polynucleotide sequence of a wild-type BBD29_14900 gene as set forth in SEQ ID NO: 1 in a host strain to cause a mutation to the base at position 1114 to provide a recombinant strain containing a mutated BBD29_14900 coding gene.

According to the method for constructing of the present invention, the engineering comprises at least one of mutagenesis, PCR site-directed mutation, and/or homologous recombination, etc.

According to the method for constructing of the present invention, the mutation refers to a change of the base at position 1114 in SEQ ID NO: 1 from guanine (G) to adenine (A). Specifically, the polynucleotide sequence containing a mutated BBD29_14900 coding gene is set forth in SEQ ID NO: 2.

Further, the method for constructing comprises the steps of:
(1) engineering a nucleotide sequence of a wild-type BBD29_14900 gene as set forth in SEQ ID NO: 1 to cause a mutation to the base at position 1114 to provide a mutated BBD29_14900 gene polynucleotide sequence;
(2) linking the mutated polynucleotide sequence to a plasmid to construct a recombinant vector; and
(3) introducing the recombinant vector into a host strain to provide a recombinant strain containing a mutated BBD29_14900 coding gene.

According to the method for constructing of the present invention, the step (1) comprises constructing a BBD29_14900 gene with a point mutation: according to a genome sequence of an unmodified strain, synthesizing two pairs of primers P1, P2 and P3, P4 for amplifying a fragment of the BBD29_14900 gene, and introducing a point mutation to a wild-type BBD29_14900 gene, SEQ ID NO: 1, by virtue of PCR site-directed mutation to provide a BBD29_14900 gene nucleotide sequence with a point mutation, SEQ ID NO: 2, designated as BBD29_14900^{G1114A}.

In an embodiment of the present invention, the genome of the unmodified strain can be derived from the strain ATCC13869, the genome sequence of which can be available from the NCBI website.

In an embodiment of the present invention, in the step (1), the primers are:
P1 (SEQ ID NO: 5):
5'-CAGTGCCAAGCTTGCATGCCTGCAGGTCGACTCTAGCGCTGTGGTTATCCTCGCTG-3'
P2 (SEQ ID NO: 6): 5'-CTGGGGCGACGCGGGGATTCAAGGCGGTCG-3'
P3 (SEQ ID NO: 7): 5'-CGACCGCCTTGAATCCCCGCGTCGCCCCAG-3'
P4 (SEQ ID NO: 8):
5'-CAGCTATGACCATGATTACGAATTCGAGCTCGGTACCCTGAGTGTCACTAGGCTAGTC-3'

In an embodiment of the present invention, the PCR amplification is performed in the following manner: pre-denaturation for 5 min at 94°C, denaturation for 30 s at 94°C, annealing for 30 s at 52°C, and extension for 40 s at 72°C for 30 circles, and overextension for 10 min at 72°C.

In an embodiment of the present invention, the overlap PCR amplification is performed in the following manner: pre-denaturation for 5 min at 94°C, denaturation for 30 s at 94°C, annealing for 30 s at 52°C, and extension for 90 s at 72°C for 30 circles, and overextension for 10 min at 72°C.

According to the method for constructing of the present invention, the step (2) comprises constructing a recombinant plasmid, comprising assembling an isolated and purified BBD29_14900^{G1114A} with pK18mobsacB plasmid by virtue of NEBuider recombination system to provide a recombinant plasmid.

According to the method for constructing of the present invention, the step (3) comprises constructing a recombinant strain by transforming a recombinant plasmid to a host strain to provide a recombinant strain.

In an embodiment of the present invention, the transforming in the step (3) is by an electrotransformation method.

In an embodiment of the present invention, the host strain is ATCC 13869.

In an embodiment of the present invention, the host strain is *Corynebacterium glutamicum* CGMCC No. 21220.

In an embodiment of the present invention, the recombination is achieved by homologous recombination.

A fourth aspect of the present invention further provides a method for constructing a recombinant strain generating L-glutamic acid.

According to the present invention, the method for constructing comprises the steps of:
amplifying the upstream and downstream homology arm fragments of BBD29_14900 and the sequence of a BBD29_14900 gene coding region and a promoter region thereof, and introducing BBD29_14900 or BBD29_14900^{G1114A} gene into the genome of a host strain in a manner of homologous recombination so as to enable the strain to overexpress the BBD29_14900 or BBD29_14900^{G1114A} gene.

In an embodiment of the present invention, the primers for amplifying the upstream homology arm fragments are:
P7 (SEQ ID NO: 11):
5'-CAGTGCCAAGCTTGCATGCCTGCAGGTCGACTCTAGGACCCGCTTGCCATACGAAG-3'
P8 (SEQ ID NO: 12): 5'-AGTGGGCTGAATTTGGGCTGATCTACTCATCTGAAGAATC-3'

In an embodiment of the present invention, the primers for amplifying the downstream homology arm fragments are:
P11 (SEQ ID NO: 15): 5'-CCGAAGCGCAAAACGCTTAGTTCGTGGGCACTCTGGTTTG-3'
P12 (SEQ ID NO: 16):
5'-CAGCTATGACCATGATTACGAATTCGAGCTCGGTACCCCATAAGAAACAACCACTTCC-3'

In an embodiment of the present invention, the primers for amplifying the sequence of the gene coding region and the promoter region thereof are:
P9 (SEQ ID NO: 13): 5'-GATTCTTCAGATGAGTAGATCAGCCCAAATTCAGCCCACT-3'
P10 (SEQ ID NO: 14): 5'-CAAACCAGAGTGCCCACGAACTAAGCGTTTTGCGCTTCGG-3'

In an embodiment of the present invention, with the above-mentioned P7/P12 as primers again, amplification is performed using as templates a mixture of three fragments, the amplified upstream homology arm fragment, downstream homology arm fragment, and fragment of the sequence of the gene coding region and the promoter region thereof, to provide an integrated homology arm fragment.

In an embodiment of the present invention, the PCR system used comprises: 10 × Ex Taq Buffer 5 µL, dNTP Mixture (2.5 mM each) 4 µL, Mg²⁺ (25 mM) 4 µL, primers (10 pM) 2 µL each, and Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 120 s at 72°C (30 circles), and overextension for 10 min at 72°C.

In an embodiment of the present invention, NEBuider recombination system is used to assemble shuttle plasmid PK18mobsacB with the integrated homology arm fragment to provide an integrative plasmid.

In an embodiment of the present invention, a host strain is transfected with the integrative plasmid to introduce BBD29_14900 or BBD29_14900^{G1114A} gene into the genome of the host strain in a manner of homologous recombination.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

A fifth aspect of the present invention further provides a method for constructing a recombinant strain generating L-glutamic acid.

According to the present invention, the method for constructing comprises the steps of:
amplifying the sequence of BBD29_14900 gene coding region and a promoter region, or the sequence of BBD29_14900^{G1114A} gene coding region and a promoter region, constructing an overexpression plasmid vector, and transforming the vector into a host strain to enable the strain to overexpress BBD29_14900 or BBD29_14900^{G1114A} gene.

In an embodiment of the present invention, the primers for amplifying the sequence of the gene coding region and the promoter region thereof are:
P17 (SEQ ID NO: 21):
5'-GCTTGCATGCCTGCAGGTCGACTCTAGAGGATCCCCCAGCCCAAATTCAGCCCACT-3'
P18 (SEQ ID NO: 22):
5'-ATCAGGCTGAAAATCTTCTCTCATCCGCCAAAACCTAAGCGTTTTGCGCTTCGG-3'

In an embodiment of the present invention, the PCR system comprises: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 90 s at 72°C (30 circles), and overextension for 10 min at 72°C.

In an embodiment of the present invention, NEBuider recombination system is used to assemble shuttle plasmid pXMJ19 with BBD29_14900 or BBD29_14900^{G1114A} fragment having its own promoters to provide an overexpression plasmid.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

The recombinant strain provided in the present invention can be individually applied to fermentation for producing L-glutamic acid, and can also be in hybrid fermentation with other L-glutamic acid-producing bacteria for producing L-glutamic acid.

Another aspect of the present invention provides a method for producing L-glutamic acid, the method comprising culturing the bacterium; and obtaining L-glutamic acid from a culture.

A bacterium can be cultured in a suitable culture medium under culturing conditions as known in the art. The culture medium can comprise: carbon source, nitrogen source, trace elements, and a combination thereof. In culturing, pH of the culture can be adjusted. In addition, bubbles can be prevented from generating in culturing, for example, by using a defoamer to prevent bubbles generation. Further, gases can be injected into the culture in culturing. The gases can comprise any ones capable of maintaining an aerobic condition for the culture. In culturing, the temperature of the culture can be from 20°C to 45°C. The generated L-glutamic acid can be recovered from the culture, i.e., the culture is treated with sulfuric acid or hydrochloric acid, etc. followed by a combination of methods such as anion-exchange chromatography, condensation, crystallization, and isoelectric precipitation.

The present invention provides a protein (a mutant protein, designated as BBD29 _14900^{D372N} protein) that is obtained from a mutation of the amino acid residue at position 372 of BBD29_14900 protein from aspartic acid to asparagine;
the BBD29_14900 protein being (a1) or (a2) or (a3) as follows:
(a1) a protein set forth in SEQ ID NO: 3;
(a2) a protein derived from a bacterium and having 95% or more identity to (a1) and relating to glutamic acid production by a bacterium;
(a3) a protein derived from (a1) and obtained by subjecting the protein indicated in (a1) to substitution and/or deletion and/or addition of one or several amino acid residues and relating to glutamic acid production by a bacterium.

The term "identity" as used herein refers to sequence similarity to a natural amino acid sequence. Identity can be evaluated with naked eyes or by computer software. In a case where computer software is utilized, the identity between two or more sequences can be represented by percentage (%) which can be used to evaluate the identity between relevant sequences.

The 95% or more identity can be, specifically, 96% or more identity, 97% or more identity, 98% or more identity, or 99% or more identity.

Specifically, the BBD29 _14900^{D372N} protein is set forth in SEQ ID NO: 4.

The BBD29_14900 gene is a gene encoding the BBD29_14900 protein.

Specifically, the BBD29_14900 gene is (b1) or (b2) or (b3) as follows:
(b1) a DNA molecule having a coding region as set forth in SEQ ID NO: 1;
(b2) a DNA molecule derived from a bacterium and having 95% or more identity to (b1) and encoding the protein;
(b3) a DNA molecule hybridized with (b1) under stringent conditions and encoding the protein.

The term "identity" as used herein refers to sequence similarity to a natural nucleic acid sequence. Identity can be evaluated with naked eyes or by computer software. In a case where computer software is utilized, the identity between two or more sequences can be represented by percentage (%) which can be used to evaluate the identity between relevant sequences.

The 95% or more identity can be, specifically, 96% or more identity, 97% or more identity, 98% or more identity, or 99% or more identity.

The stringent conditions can be hybridizing at 65°C in a solution of 0.1 × SSPE (or 0.1 × SSC), 0.1% SDS, and washing membrane.

The coding gene (designated as BBD29_14900^{G1114A} gene) of the BBD29_14900^{D372N} protein also falls into the scope of protection of the present invention.

Specifically, the BBD29_14900^{G1114A} gene is (c1) or (c2) or (c3) as follows:
(c1) a DNA molecule having a coding region as set forth in SEQ ID NO: 2;
(c2) a DNA molecule derived from a bacterium and having 95% or more identity to (b1) and encoding the protein;
(c3) a DNA molecule hybridized with (c1) under stringent conditions and encoding the protein.

The term "identity" as used herein refers to sequence similarity to a natural nucleic acid sequence. Identity can be evaluated with naked eyes or by computer software. In a case where computer software is utilized, the identity between two or more sequences can be represented by percentage (%) which can be used to evaluate the identity between relevant sequences.

The 95% or more identity can be, specifically, 96% or more identity, 97% or more identity, 98% or more identity, or 99% or more identity.

The stringent conditions can be hybridizing at 65°C in a solution of 0.1 × SSPE (or 0.1 × SSC), 0.1% SDS, and washing membrane.

A DNA molecule having BBD29_14900^{G1114A} gene, an expression cassette having BBD29_14900^{G1114A} gene, or a recombinant vector having BBD29_14900^{G1114A} gene, or a recombinant bacterium having BBD29_14900^{G1114A} gene each falls into the scope of protection of the present invention.

Illustratively, a DNA molecule having BBD29_14900^{G1114A} gene can be a DNA molecule set forth in SEQ ID NO: 30 or a DNA molecule set forth in SEQ ID NO: 31.

Illustratively, a recombinant vector having BBD29_14900^{G1114A} gene can be a plasmid having a DNA molecule set forth in SEQ ID NO: 30 or a plasmid having a DNA molecule set forth in SEQ ID NO: 31.

Illustratively, a recombinant vector having BBD29_14900^{G1114A} gene can be an integrative plasmid PK18mobsacB-BBD29_14900^{G1114A} or an overexpression plasmid pXMJ19-BBD29_14900^{G1114A} in Examples.

The recombinant bacterium can be, specifically, a recombinant bacterium.

Illustratively, a recombinant bacterium having BBD29_14900^{G1114A} gene can be a recombinant bacterium having a DNA molecule set forth in SEQ ID NO: 30, or a recombinant bacterium having a DNA molecule set forth in SEQ ID NO: 31, or a recombinant bacterium having a DNA molecule set forth in SEQ ID NO: 2.

A recombinant bacterium having BBD29_14900^{G1114A} gene can be prepared, specifically, by substituting the BBD29_14900 gene in the genome of a bacterium with the BBD29_14900^{G1114A} gene.

Illustratively, substituting the BBD29_14900 gene in the genome of a bacterium with the BBD29_14900^{G1114A} gene is implemented by introducing a DNA molecule set forth in SEQ ID NO: 29 into the bacterium.

Illustratively, substituting the BBD29_14900 gene in the genome of a bacterium with the BBD29_14900^{G1114A} gene is implemented by introducing the vector pK18-BBD29_14900^{G1114A} in Examples into the bacterium.

In the recombinant bacterium, the BBD29_14900^{G1114A} gene can be integrated into genomic DNA for expression, or can be expressed in a plasmid.

Illustratively, a recombinant bacterium having BBD29_14900^{G1114A} gene can be prepared, specifically, by introducing a DNA molecule set forth in SEQ ID NO: 30 or a DNA molecule set forth in SEQ ID NO: 31 into a bacterium.

Illustratively, a recombinant bacterium having BBD29_14900^{G1114A} gene can be prepared, specifically, by introducing a plasmid having a DNA molecule set forth in SEQ ID NO: 30 or a plasmid having a DNA molecule set forth in SEQ ID NO: 31 into a bacterium.

Illustratively, a recombinant bacterium having BBD29_14900^{G1114A} gene can be prepared, specifically, by introducing the integrative plasmid PK18mobsacB-BBD29_14900^{G1114A} or the overexpression plasmid pXMJ19-BBD29-14900^{G1114A} in Examples into a bacterium.

The present invention also protects use of BBD29_14900^{D372N} protein, BBD29_14900^{G1114A} gene, an expression cassette having BBD29_14900^{G1114A} gene, or a recombinant vector having BBD29_14900^{G1114A} gene, or a recombinant bacterium having BBD29_14900^{G1114A} gene;

The use is (I) or (II) as follows:
(I) use in increasing glutamic acid production in a bacterium;
(II) use in the production of glutamic acid.

The present invention also protects use of a particular substance;
the use being (I) or (II) as follows:
(I) use in increasing glutamic acid production by a bacterium;
(II) use in the production of glutamic acid.

The particular substance is (d1), (d2), (d3), (cd4), (d5) or (d6) as follows:
(d1) a substance for increasing the expression of BBD29_14900^{G1114A} gene;
(d2) a substance for increasing the abundance of BBD29_14900^{D372N} protein;
(d3) a substance for increasing the activity of BBD29_14900^{D372N} protein;
(d4) a substance for increasing the expression of BBD29_14900 gene;
(d5) a substance for increasing the abundance of BBD29_14900 protein;
(d6) a substance for increasing the activity of BBD29_14900 protein.

Illustratively, the substance for increasing the expression of the BBD29_14900^{G1114A} gene can be, specifically, BBD29_14900^{G1114A} gene or a recombinant plasmid having BBD29_14900^{G1114A} gene. Illustratively, the recombinant plasmid can be the integrative plasmid PK18mobsacB-BBD29_14900^{G1114A} or the overexpression plasmid pXMJ19-BBD29_14900^{G1114A} in Examples.

Illustratively, the substance for increasing the expression of the BBD29_14900 gene can be, specifically, BBD29_14900 gene or a recombinant plasmid having BBD29_14900 gene. Illustratively, the recombinant plasmid can be the integrative plasmid PK18mobsacB-BBD29_14900 or the overexpression plasmid pXMJ19-BBD29_14900 in Examples.

The present invention further provides a recombinant bacterium that is obtained by overexpressing the BBD29_14900^{G1114A} gene or BBD29_14900 gene in a bacterium.

Illustratively, overexpressing the BBD29_14900^{G1114A} gene is implemented by introducing into a bacterium the BBD29_14900^{G1114A} gene or a recombinant plasmid having BBD29_14900^{G1114A} gene. Illustratively, the recombinant plasmid can be the integrative plasmid PK18mobsacB-BBD29_14900^{G1114A} or the overexpression plasmid pXMJ19-BBD29_14900^{G1114A} in Examples.

Illustratively, overexpressing the BBD29_14900 gene is implemented by introducing into a bacterium the BBD29_14900 gene or a recombinant plasmid having BBD29_14900 gene. Illustratively, the recombinant plasmid can be the integrative plasmid PK18mobsacB-BBD29_14900 or the overexpression plasmid pXMJ19-BBD29_14900 in Examples.

The present invention also protects use of the recombinant bacterium in the preparation of glutamic acid.

The present invention also protects a method for increasing the production of glutamic acid of a bacterium, comprising the step of substituting the BBD29_14900 gene in the genome of a bacterium with the BBD29_14900^{G1114A} gene.

Illustratively, substituting the BBD29_14900 gene in the genome of a bacterium with the BBD29_14900^{G1114A} gene is implemented by introducing the vector pK18-BBD29_14900^{G1114A} in Examples into a bacterium.

The present invention also provides a method for increasing the production of glutamic acid of a bacterium, comprising the step of: overexpressing the BBD29_14900^{G1114A} gene in a bacterium or overexpressing the BBD29_14900 gene in a bacterium, or increasing the abundance of the BBD29_14900^{D372N} protein in a bacterium or increasing the abundance of the BBD29_14900 protein in a bacterium, or increasing the activity of the BBD29_14900^{D372N} protein in a bacterium or increasing the activity of the BBD29_14900 protein in a bacterium.

Illustratively, overexpressing the BBD29_14900^{G1114A} gene is implemented by introducing into a bacterium the BBD29_14900^{G1114A} gene or a recombinant plasmid having BBD29_14900^{G1114A} gene. Illustratively, the recombinant plasmid can be the integrative plasmid PK18mobsacB-BBD29_14900^{G1114A} or the overexpression plasmid pXMJ19-BBD29_14900^{G1114A} in Examples.

Illustratively, overexpressing the BBD29_14900 gene is implemented by introducing into a bacterium the BBD29_14900 gene or a recombinant plasmid having BBD29_14900 gene. Illustratively, the recombinant plasmid can be the integrative plasmid PK18mobsacB-BBD29_14900 or the overexpression plasmid pXMJ19-BBD29_14900 in Examples.

The present invention also protects use of BBD29_14900^{D372N} protein or BBD29_14900 protein in the regulation of the production of glutamic acid of a bacterium.

The regulation is a positive regulation, i.e., an increased content of the BBD29 _14900^{D372N} protein for an increased production of glutamic acid.

The regulation is a positive regulation, i.e., a reduced content of the BBD29 _14900^{D372N} protein for a reduced production of glutamic acid.

The regulation is a positive regulation, i.e., an increased content of the BBD29_14900 protein for an increased production of glutamic acid.

The regulation is a positive regulation, i.e., a reduced content of the BBD29_14900 protein for a reduced production of glutamic acid.

In the use of the recombinant bacterium for the preparation of glutamic acid, a specific method comprises the step of fermenting the recombinant bacterium.

Those skilled in the art can adopt a fermentation method in the prior art for fermentation, or the fermentation method can be optimized and improved through routine experimentation. Fermentation with a bacterium can be done in a suitable culture medium under fermentation conditions as known in the art. The culture medium can comprise: carbon source, nitrogen source, trace elements, and a combination thereof. In culturing, pH of the culture can be adjusted. In addition, bubbles can be prevented from generating in culturing, for example, by using a defoamer to prevent bubbles generation. Further, gases can be injected into the culture in culturing. The gases can comprise any ones capable of maintaining an aerobic condition for the culture. In culturing, the temperature of the culture can be from 20°C to 45°C.

The method further comprises the step of obtaining glutamic acid from the culture. Obtaining glutamic acid from the culture can be implemented in various ways, including but not limited to, treatment of the culture with sulfuric acid or hydrochloric acid, etc. followed by a combination of methods such as anion-exchange chromatography, condensation, crystallization, and isoelectric precipitation.

In the fermentation, an illustrative formulation of the culture medium for fermentation is seen in Table 3, with water as balance.

In the fermentation, an illustrative process for the control of fermentation is seen in Table 4.

Illustratively, in the fermentation, the bacterium is at a concentration of 15 g/L in the system.

Illustratively, in the fermenting process of the fermentation: sugar content in the system (residual sugar) is controlled by supplementing an aqueous solution of glucose.

Any one of the above bacteria includes, but is not limited to: a bacterium of the genus *Corynebacterium,* preferably, *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis.*

Any one of the above bacteria is a bacterium having capacity of producing glutamic acid.

"Bacterium having capacity of producing glutamic acid" refers to a bacterium having capacity of producing and accumulating glutamic acid in a culture medium and/or a cell of the bacterium. Thereby, glutamic acid can be collected when the bacterium is cultured in the culture medium.

The bacterium can be a wild-type bacterium harvested in nature, or can be a modified bacterium.

"Modified bacterium" refers to an engineered bacterium obtained by performing artificial mutation and/or mutagenesis on a wild-type bacterium harvested in nature.

Specifically, the *Corynebacterium glutamicum* can be *Corynebacterium glutamicum* CGMCC21260.

*Corynebacterium glutamicum* CGMCC21220, with YPGLU001 as its strain name, abbreviated as *Corynebacterium glutamicum* CGMCC21220, has been deposited in China General Microbiological Culture Collection Center (abbreviated as CGMCC, Address: No. 3, Yard 1, Beichen West Road, Chaoyang District, Beijing, Institute of Microbiology, Chinese Academy of Sciences) on November 23, 2020, with Accession No. CGMCC 21220.

Any one of the above glutamic acids is to be understood as glutamic acid in the generic sense, including glutamic acid in a free form, a salt of glutamic acid, or a mixture of both.

Specifically, the glutamic acid is L-glutamic acid.

Any of the above methods or uses can also be applied to the preparation of a downstream product from glutamic acid.

The BBD29_14900 protein in *Corynebacterium glutamicum* is set forth in SEQ ID NO: 3, with a coding gene thereof set forth in SEQ ID NO: 1. The present invention provides a BBD29 _14900^{D372N} protein set forth in SEQ ID NO: 4 by introducing a point mutation, with a coding gene of the BBD29_14900^{D372N} protein set forth in SEQ ID NO: 2. Compared with the BBD29_14900 gene, the BBD29_14900^{G1114A} gene differs in a change at position 1114 from guanine deoxyribonucleotide (G) to adenine deoxyribonucleotide (A). Compared with the BBD29_14900 protein, the BBD29_14900^{D372N} protein differs in a change in the amino acid residue at position 372 from aspartic acid (D) to asparagine (N).

The present invention discovers that BBD29_14900 protein has a positive regulation over the production of glutamic acid of a bacterium, i.e., an increased content of BBD29_14900 protein for an increased production of glutamic acid; a reduced content of BBD29_14900 protein for a reduced production of glutamic acid. Inhibiting the expression of BBD29_14900 gene can reduce the production of glutamic acid, while overexpressing BBD29_14900 gene increases the production of glutamic acid. Further, the present invention provides a BBD29_14900^{D372N} protein from a point mutation, which is functionally superior to the BBD29_14900 protein. The present invention is of great application value for industrialized production of glutamic acid.

### Best Modes for Carrying out the Invention

The following examples are provided to facilitate a better understanding of the present invention and not to limit the present invention. Unless otherwise stated specially, the experimental methods in the following examples are all routine methods. Unless otherwise stated specially, the experimental materials used in the following examples are all available from conventional stores for biochemical reagents.

The culture media used for culturing strains in the following examples are all obtained by adding other ingredients on the basis of a basic medium. Other ingredients are sucrose, kanamycin or chloramphenicol, etc. Agarose is contained in a solid medium. Components in a basic medium are seen in Table 1. Unless otherwise stated specially, strains are all cultured at a temperature of 32°C in Examples.

**Table 1**

| Ingredients | Formulation |
|---|---|
| Sucrose | 10 g/L |
| Polypeptone | 10 g/L |
| Beef Extract | 10 g/L |
| Powdered Yeast | 5 g/L |
| Urea | 2 g/L |
| Sodium Chloride | 2.5 g/L |
| Powdered Agar | 20 g/L |
| pH | 7.0 |

The formulation for PAGE in sscp electrophoresis is seen in Table 2. Conditions for electrophoresis: placing an electrophoresis tank in ice, with 1 × TBE buffer, for electrophoresis at a voltage of 120 V for 10 h.

**Table 2**

| Ingredients | Amount (Final Concentration of 8% for Acrylamide Formulation) |
|---|---|
| 40% Acrylamide | 8 mL |
| ddH₂O | 26 mL |
| Glycerol | 4 mL |
| 10 × TBE | 2 mL |
| TEMED | 40 µL |
| 10% APS | 600 µL |

*Corynebacterium glutamicum* CGMCC21220, with YPGLU001 as its strain name, abbreviated as *Corynebacterium glutamicum* CGMCC21220, has been deposited in China General Microbiological Culture Collection Center (abbreviated as CGMCC, Address: No. 3, Yard 1, Beichen West Road, Chaoyang District, Beijing, Institute of Microbiology, Chinese Academy of Sciences) on November 23, 2020, with Accession No. CGMCC 21220. *Corynebacterium glutamicum* ATCC 13869, i.e., *Corynebacterium glutamicum* CICC 20216; CICC is short for China Center of Industrial Culture Collection.

### Example 1. Construction of Transformation Vector pK18-BBD29_14900^{G1114A} Comprising BBD29_14900 Gene Coding Region with Point Mutation

According to the genome sequence of *Corynebacterium glutamicum* ATCC13869 published on NCBI, two pairs of primers for amplifying the sequence of BBD29_14900 gene coding region are designed and synthesized, and a point mutation is introduced into *Corynebacterium glutamicum* CGMCC21220 in allelic replacement (the BBD29_14900 gene coding region on the chromosome of *Corynebacterium glutamicum* CGMCC21220 is confirmed consistent with that on the chromosome of *Corynebacterium glutamicum* ATCC13869 following sequencing). The amino acid sequence corresponding to a coded protein before mutation is SEQ ID NO: 3, and the nucleotide sequence of BBD29_14900 gene before mutation is SEQ ID NO: 1. A single point mutation is introduced to the gene, i.e., a change of guanine deoxyribonucleotide (G) at position 1114 in the coding region to adenine deoxyribonucleotide (A). Correspondingly, a single point mutation is caused to the protein, i.e., a change of the amino acid residue at position 372 from aspartic acid (D) to asparagine (N). The mutated gene is designated as BBD29_14900^{G1114A} gene as set forth in SEQ ID NO: 2. The mutated protein is designatedas BBD29_14900^{D372N} protein as set forth in SEQ ID NO: 4.

The primers are as follows (synthesized by Invitrogen Corporation, Shanghai):
P1 (SEQ ID NO: 5):
5'-CAGTGCCAAGCTTGCATGCCTGCAGGTCGACTCTAGCGCTGTGGTTATCCTCGCTG-3'
P2 (SEQ ID NO: 6): 5'-CTGGGGCGACGCGGGGATTCAAGGCGGTCG-3'
P3 (SEQ ID NO: 7): 5'-CGACCGCCTTGAATCCCCGCGTCGCCCCAG-3'
P4 (SEQ ID NO: 8):
5'-CAGCTATGACCATGATTACGAATTCGAGCTCGGTACCCTGAGTGTCACTAGGCTAGTC-3'
1. PCR amplification is performed using *Corynebacterium glutamicum* ATCC 13869 as a template and using a pair of primers consisting of P1 and P2, or a pair of primers consisting of P3 and P4, respectively. PCR system (50 µL): a template, 10 × Ex Taq Buffer 5 µL, dNTP Mixture (2.5 mM each) 4 µL, Mg²⁺ (25 mM) 4 µL, primers (10 µM) 2 µL each, Ex Taq (5 U/µL) 0.25 µL, with water as balance.
   The PCR amplification is performed by pre-denaturation for 5 min at 94°C, denaturation for 30 s at 94°C, annealing for 30 s at 52°C, and extension for 40 s at 72°C for 30 circles, and overextension for 10 min at 72°C.
2. The amplified products in the two PCR systems in step 1 are recovered, respectively, which in the meantime are used as a template for PCR amplification using a pair of primers consisting of P1 and P4. PCR system (50 µL): a template, 10 × Ex Taq Buffer 5 µL, dNTP Mixture (2.5 mM each) 4 µL, Mg²⁺ (25 mM) 4 µL, primers (10 µM) 2 µL each, Ex Taq (5 U/µL) 0.25 µL, with water as balance.
   The PCR amplification is performed by pre-denaturation for 5 min at 94°C, denaturation for 30 s at 94°C, annealing for 30 s at 52°C, and extension for 90 s at 72°C for 30 circles, and overextension for 10 min at 72°C.
3. The amplified products in about 1253 bp obtained from the PCR amplification in step 2 are recovered, and the recovered DNA fragment is BBD29_14900^{G1114A} fragment (the DNA fragment is set forth in SEQ ID NO: 29 upon sequencing).
   The BBD29_14900^{G1114A} fragment can be integrated into *Corynebacterium glutamicum* CGMCC21220 through homologous recombination to cause a mutation of the nucleotide at position 1114 of the BBD29_14900 gene coding region from guanine deoxyribonucleotide (G) to adenine deoxyribonucleotide (A), thereby finally causing a mutation of the amino acid residue at position 372 of the coded protein from aspartic acid (D) to asparagine (N).
4. Restriction endonuclease *Xba I* is employed for cleaving pK18mobsacB plasmid (a product from Addgene Corporation) to recover a linearized plasmid.
5. The resulting BBD29_14900^{G1114A} fragment from step 3 and the resulting linearized plasmid from step 4 are assembled by virtue of NEBuider recombination system to provide vector pK18-BBD29_14900^{G1114A}. Vector pK18-BBD29_14900^{G1114A} has been verified by sequencing, having the BBD29_14900^{G1114A} fragment as set forth in SEQ ID NO: 29 therein. Vector pK18-BBD29_14900^{G1114A} has a kanamycin-resistant marker.

### Example 2. Construction of Engineered Strain Comprising BBD29_14900^{G1114A} with Point Mutation

The vector pK18-BBD29_14900^{G1114A} constructed in Example 1 is introduced into *Corynebacterium glutamicum* CGMCC21220 through electrotransformation, and the cultured mono-colonies are each identified through PCR with a pair of primers consisting of primer P1 and universal primer M13R (M13R: 5'-CAGGAAACAGCTATGACC-3') for a positive strain with a band in about 1260 bp following amplification. The positive strain is cultured on a culture medium plate containing 15% sucrose, and the cultured mono-colonies are cultured on culture medium plates with and without kanamycin, respectively. Strains that grow on a culture medium without kanamycin and do not grow on a culture medium with kanamycin are further subjected to PCR for identification with a pair of primers consisting of P5 and P6 (synthesized by Invitrogen Corporation, Shanghai). The PCR amplified products from PCR identification are recovered and subjected to denaturation at a high temperature and ice bath before sscp electrophoresis (with an amplified fragment of vector pK18-BBD29_14900^{G1114A} as a positive control, an amplified fragment of *Corynebacterium glutamicum* ATCC13869 as a negative control, and water as a blank control). Since fragments are different in their structures, their locations in electrophoresis are different. Thus, a strain having successful allelic replacement is such a strain that the location of its fragment in electrophoresis is inconsistent with that of a negative control fragment and consistent with that of a positive control fragment. PCR amplification is performed on the screened strain having successful allelic replacement with a pair of primers consisting of primers P5 and P6 to recover the amplified product for linking to vector PMD19-T, followed by sequencing. The strain having successful allelic replacement having been verified by sequence alignment is designated as *Corynebacterium glutamicum* YPG-019.
P5 (SEQ ID NO: 9): 5'-GCAGCCAAGGCCAAGAAGAT-3';
P6 (SEQ ID NO: 10): 5'-TCCCTGTTTAAGACTGCATT-3'.

Compared with *Corynebacterium glutamicum* CGMCC21220, *Corynebacterium glutamicum* YPG-019 differs only in the substitution of the BBD29_14900 gene as set forth in SEQ ID NO: 1 in the Sequence Listing with the BBD29_14900^{G1114A} gene as set forth in SEQ ID NO: 2 in the Sequence Listing in the genome of *Corynebacterium glutamicum* CGMCC21220. SEQ ID NO: 1 differs from SEQ ID NO: 2 only in one nucleotide, at position 1114.

### Example 3. Construction of Engineered Strain with BBD29_14900 or BBD29_14900^{G1114A} Gene Overexpressing on Genome

### I. Construction of Integrative Plasmid

According to the genome sequence of *Corynebacterium glutamicum* ATCC 13869 published on NCBI, three pairs of primers are designed and synthesized for respective amplification of upstream homology arm fragment, BBD29_14900 gene coding region (or BBD29_14900^{G1114A} gene coding region), and downstream homology arm fragment for integration of BBD29_14900 gene or BBD29_14900^{G1114A} gene into genomic DNA of *Corynebacterium glutamicum* CGMCC21220 through homologous recombination.

The primers are as follows (synthesized by Invitrogen Corporation, Shanghai):
P7 (SEQ ID NO: 11):
5'-CAGTGCCAAGCTTGCATGCCTGCAGGTCGACTCTAGGACCCGCTTGCCATACGAAG-3'
P8 (SEQ ID NO: 12): 5'-AGTGGGCTGAATTTGGGCTGATCTACTCATCTGAAGAATC-3'
P9 (SEQ ID NO: 13): 5'-GATTCTTCAGATGAGTAGATCAGCCCAAATTCAGCCCACT-3'
P10 (SEQ ID NO: 14): 5'-CAAACCAGAGTGCCCACGAACTAAGCGTTTTGCGCTTCGG-3'
P11 (SEQ ID NO: 15): 5'-CCGAAGCGCAAAACGCTTAGTTCGTGGGCACTCTGGTTTG-3'
P12 (SEQ ID NO: 16):
5'-CAGCTATGACCATGATTACGAATTCGAGCTCGGTACCCCATAAGAAACAACCACTTCC-3'

Using *Corynebacterium glutamicum* YPG-019 as a template, PCR amplification is performed with a pair of primers consisting of P7/P8, a pair of primers consisting of P9/P10, and a pair of primers consisting of P11/P12, respectively, to provide upstream homology arm fragment in about 806 bp, BBD29_14900^{G1114A} gene fragment in about 1494 bp, and downstream homology arm fragment in about 788 bp. Then, amplification is further performed with a pair of primers consisting of P7/P12 using a mixture of the above three amplified fragments as a template to provide an integrated homology arm fragment in about 3008 bp (as set forth in SEQ ID NO: 30 upon sequencing). NEBuider recombination system is used to link the integrated homology arm fragment to the shuttle plasmid PK18mobsacB having been cleaved with *Xba I* and recovered to provide an integrative plasmid PK18mobsacB-BBD29_14900^{G1114A}. The integrative plasmid contains a kanamycin-resistant marker, and a recombinant with the plasmid integrated into the genome is obtained by screening with kanamycin. PCR system (50 µL): a template, 10 × Ex Taq Buffer 5 µL, dNTP Mixture (2.5 mM each) 4 µL, Mg²⁺ (25 mM) 4 µL, primers (10 µM) 2 µL each, Ex Taq (5 U/µL) 0.25 µL, with water as balance. The PCR amplification is performed by pre-denaturation for 5 min at 94°C, denaturation for 30 s at 94°C, annealing for 30 s at 52°C, and extension for 120 s at 72°C for 30 circles, and overextension for 10 min at 72°C.

The above steps are performed with *Corynebacterium glutamicum* ATCC13869 in place of *Corynebacterium glutamicum* YPG-019 to provide an integrative plasmid PK18mobsacB-BBD29_14900.

### II. Preparation of Recombinant Bacterium

The integrative plasmid is introduced into *Corynebacterium glutamicum* CGMCC21220 by virtue of electrotransformation, and PCR identification is performed on the cultured mono-colonies with a pair of primers consisting of P13/P14 for a positive strain having been successfully transformed and containing a fragment in about 1827 bp, while a strain without any fragments amplified therefrom is an unsuccessfully transformed one. Having being screened with 15% sucrose, the positive strain is cultured on culture medium plates with and without kanamycin, respectively, and strains that grow on a culture medium without kanamycin and do not grow on a culture medium with kanamycin are further subjected to PCR for identification with a pair of primers consisting of P15/P16. The strain with a fragment amplified therefrom in about 1517 bp is a strain with a target gene integrated into the genome of *Corynebacterium glutamicum* CGMCC21220.
P13 (SEQ ID NO: 17): 5'-GTCCAAGGTGACGGCCGCAC-3'
P14 (SEQ ID NO: 18): 5'-GCAGCCTTAACTGGGGAAAG-3'
P15 (SEQ ID NO: 19): 5'-GGAGCGCCGCCTCATCGAGC-3'
P16 (SEQ ID NO: 20): 5'-ATATTCGGCCCAGCAGCAGC-3'

The integrative plasmid PK18mobsacB-BBD29_14900 is subjected to the above steps to provide a recombinant bacterium as *Corynebacterium glutamicum* YPG-020. *Corynebacterium glutamicum* YPG-020 is a recombinant bacterium with BBD29_14900 gene overexpressing in genomic DNA.

The integrative plasmid PK18mobsacB-BBD29_14900^{G1114A} is subjected to the above steps to provide a recombinant bacterium as *Corynebacterium glutamicum* YPG-021. *Corynebacterium glutamicum* YPG-021 is a recombinant bacterium with BBD29_14900^{G1114A} gene overexpressing in genomic DNA.

### Example 4. Construction of Engineered Strain with BBD29_14900 or BBD29_14900^{G1114A} Gene Overexpressing on Plasmid

### I. Construction of Recombinant Plasmid

According to the genome sequence of a wild-type *Corynebacterium glutamicum* ATCC 13869 published on NCBI, a pair of primers, P17 and P18 (synthesized by Invitrogen Corporation, Shanghai), are designed and synthesized for amplifying the sequence of BBD29_14900 gene coding region and promoter region.
P17 (SEQ ID NO: 21):
5'-GCTTGCATGCCTGCAGGTCGACTCTAGAGGATCCCCCAGCCCAAATTCAGCCCACT-3'
P18 (SEQ ID NO: 22):
5'-ATCAGGCTGAAAATCTTCTCTCATCCGCCAAAACCTAAGCGTTTTGCGCTTCGG-3'

Using *Corynebacterium glutamicum* YPG-019 as a template, PCR amplification is performed with a pair of primers consisting of primers P17/P18 to provide a BBD29_14900^{G1114A} gene fragment in 1524 bp (the BBD29_14900^{G1114A} gene fragment is set forth in SEQ ID NO: 31 upon sequencing). NEBuider recombination system is used to link the BBD29_14900^{G1114A} gene fragment to the shuttle plasmid pXMJ19 having been cleaved with *Eco*R I and recovered to provide an overexpression plasmid pXMJ19-BBD29_14900^{G1114A}. The overexpression plasmid contains a chloramphenicol-resistant marker, and the transformation of the plasmid into the strain can be achieved by screening with chloramphenicol. PCR system (50 µL): a template, 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, with water as balance. The PCR amplification is performed by pre-denaturation for 5 min at 94°C, degeneration for 30 s at 94°C, annealing for 30 s at 52°C, and extension for 90 s at 72°C for 30 circles, and overextension for 10 min at 72°C.

The above steps are performed with *Corynebacterium glutamicum* YPG-020 in place of *Corynebacterium glutamicum* YPG-019 to provide an overexpression plasmid pXMJ19-BBD29_14900.

### II. Preparation of Recombinant Bacterium

The overextension plasmid is introduced into *Corynebacterium glutamicum* CGMCC21220 by virtue of electrotransformation, and PCR identification is performed on the cultured mono-colonies with a pair of primers consisting of M13R (-48) and P18. The strain with a fragment amplified therefrom by PCR in about 1536 bp is a recombinant bacterium. M13R (-48): 5'-AGCGGATAACAATTTCACACAGGA-3'.

The overexpression plasmid pXMJ19-BBD29_14900 is subjected to the above steps to provide a recombinant bacterium as *Corynebacterium glutamicum* YPG-022. *Corynebacterium glutamicum* YPG-022 is a recombinant bacterium with a plasmid overexpressing BBD29_14900 gene.

The overexpression plasmid pXMJ19-BBD29_14900^{G1114A} is subjected to the above steps to provide a recombinant bacterium as *Corynebacterium glutamicum* YPG-023. *Corynebacterium glutamicum* YPG-023 is a recombinant bacterium with a plasmid overexpressing BBD29_14900^{G1114A} gene.

### Example 5. Construction of Engineered Strain with BBD29_14900 Gene Deleted from Genome

### I. Construction of Knockout Plasmid

According to the genome sequence of *Corynebacterium glutamicum* ATCC 13869 published on NCBI, two pairs of primers for amplifying fragments at both ends of the BBD29_14900 gene coding region are synthesized for amplifying upstream and downstream homology arm fragments.

The primers are as follows (synthesized by Invitrogen Corporation, Shanghai):
P19 (SEQ ID NO: 23):
5'-CAGTGCCAAGCTTGCATGCCTGCAGGTCGACTCTAGTCGCTGAAACAGCAGGGGAC-3'
P20 (SEQ ID NO: 24): 5'-AGGCGTCGATAAGCAAATTTATCATTTAGCCTTGTTAATC-3'
P21 (SEQ ID NO: 25): 5'-GATTAACAAGGCTAAATGATAAATTTGCTTATCGACGCCT-3'
P22 (SEQ ID NO: 26):
5'-CAGCTATGACCATGATTACGAATTCGAGCTCGGTACCCAACTTGCCATGAGTCGTCTT-3'

Using *Corynebacterium glutamicum* ATCC13869 as a template, PCR amplification is performed with a pair of primers consisting of P19/P20 or a pair of primers consisting of P21/P22, respectively, to provide an upstream homology arm fragment (in about 804 bp) and a downstream homology arm fragment (in about 791 bp), respectively. Meanwhile, using the upstream homology arm fragment and the downstream homology arm fragment as a template, PCR amplification is performed with a pair of primers consisting of primers P19/P22 to provide an integral homology arm fragment (in about 1555 bp, as set forth in SEQ ID NO: 30 upon sequencing). NEBuider recombination system is used to link the integral homology arm fragment to the shuttle plasmid pk18mobsacB having been cleaved with *Xba* I and recovered to provide a knockout plasmid. The knockout plasmid contains a kanamycin-resistant marker.

### II. Preparation of Recombinant Bacterium

The knockout plasmid is introduced into *Corynebacterium glutamicum* CGMCC21220 by virtue of electrotransformation, and PCR identification is performed on the cultured mono-colonies each with a pair of primers consisting of P23 and P24 (synthesized by Invitrogen Corporation, Shanghai) for a positive strain having been successfully transformed, with bands in about 1481 bp and 2660 bp, while the strain with a band in 1481 bp alone is an unsuccessfully transformed one. Having being screened on a culture medium with 15% sucrose, the positive strain is cultured on culture medium plates with and without kanamycin, respectively, and strains that grow on a culture medium without kanamycin and do not grow on a culture medium with kanamycin are further subjected to PCR for identification with a pair of primers consisting of P23 and P24. The strain with only one product amplified therefrom and sized in 1481 bp is a genetically engineered strain with the BBD29_14900 gene coding region knocked out, designated as *Corynebacterium glutamicum* YPG-024.
P23 (SEQ ID NO: 27): 5'-TCGCTGAAACAGCAGGGGAC-3'
P24 (SEQ ID NO: 28): 5'-AACTTGCCATGAGTCGTCTT-3'

### Example 6. Experiments on Fermentation of L-glutamic acid

Each recombinant bacterium and *Corynebacterium glutamicum* CGMCC21220 constructed in the above Examples are subjected to fermentation in a fermentation tank, BLBIO-5GC-4-H type (purchased from Shanghai Bailun Biological Technology Co., Ltd.).

Formulation of the culture medium for fermentation is seen in Table 3 (with water as balance) in which d1 means 0.1 L.

Control process is seen in Table 4. At the initial moment when seeding is completed, the bacterium is at a concentration of 15 g/L in the system. During the fermentation: sugar content in the system (residual sugar) is controlled by supplementing an aqueous solution containing 50-55% glucose.

Triplicates are set for treatment each. Results are seen in Table 5.

**Table 3 Formulation of Culture Medium for Fermentation**

| Name of Reagent | Compounding Ratio |
|---|---|
| Glucose | 5.0 g/L |
| Phosphoric Acid | 0.38 g/L |
| Magnesium Sulfate | 1.85 g/L |
| Potassium Chloride | 1.6 g/L |
| Biotin | 550 µg/L |
| Vitamin B1 | 300 µg/L |
| Ferrous Sulfate | 10 mg/L |
| Manganese Sulfate | 10 g/dl |
| KH₂PO₄ | 2.8 g/L |
| Vitamin C | 0.75 mg/L |
| Vitamin B12 | 2.5 µg/L |
| Para-Aminobenzoic Acid | 0.75 mg/L |
| Defoamer | 0.0015 mL/dL |
| Betaine | 1.5 g/L |
| Cane-Sugar Molasses | 7 mL/L |
| Corn Steep Liquor | 77 mL/L |
| Aspartic Acid | 1.7 g/L |
| Hair Powder | 2 g/L |

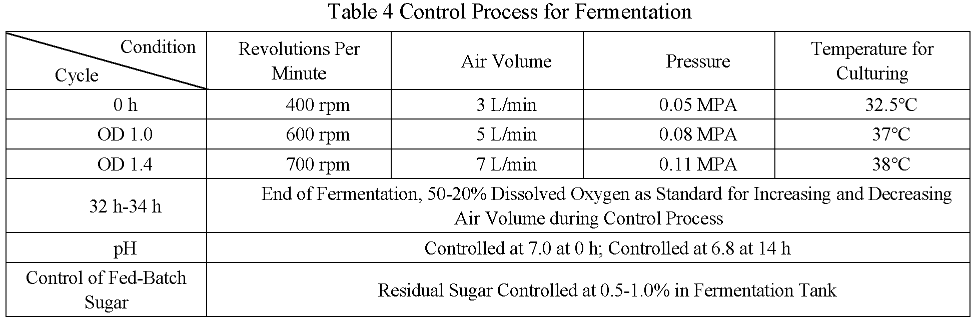

**Table 5 Results of Experiments on Fermentation of L-glutamic Acid**

| Strain | Production for L-Glutamic Acid (g/L) | OD (562 nm) |
|---|---|---|
| *Corynebacterium glutamicum* CGMCC21220 | 102.1 | 46.1 |
| **YPG-019** | 102.3 | 45.6 |
| YPG-020 | 105.1 | 46.6 |
| **YPG-021** | 106.8 | 45.4 |
| YPG-022 | 107.5 | 45.1 |
| **YPG-023** | 108.8 | 45.5 |
| YPG-024 | 96.5 | 46.3 |

Results from Table 5 show that a point mutation, BBD29_14900^{G1114A}, to and overexpression of the BBD29_14900 gene coding region in *Corynebacterium glutamicum* facilitate an increase in the production of L-glutamic acid, while weakening or knocking out the gene is adverse to the accumulation of L-glutamic acid.

The present invention has been described in detail above. For those skilled in the art, the present invention can be implemented in a wide range with equivalent parameters, concentrations and conditions without departing from the spirit and scope of the present invention and without unnecessary experiments. Although specific embodiments have been provided in the present invention, it should be understood that further improvements can be made to the present invention. In summary, according to the principles of the present invention, the present application is intended to include any variations, uses, or improvements to the present invention, including changes made with routine techniques as known in the art, which depart from the scope disclosed in the present application. Some essential features can be applied in accordance with the scope of the following appended claims.

### Industrial Application

The disclosure of the present invention introduces the following effects: the present invention has found out that, by weakening or knocking out the BBD29_14900 gene, the product coded by the gene has an effect on L-glutamic acid production capacity, and that a recombinant strain obtained by introducing a point mutation to the coding sequence, or by increasing the copy number of, or overexpressing the gene facilitates production of glutamic acid at a higher concentration as compared with an unmodified strain.

## Claims

1. A bacterium for generating L-glutamic acid, **characterized in** having an improved expression of a polynucleotide encoding an amino acid sequence of SEQ ID NO: 3 or a homologous sequence thereof;
preferably, the improved expression is an enhanced expression of the polynucleotide encoding an amino acid sequence of SEQ ID NO: 3 or a homologous sequence thereof, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

2. The bacterium of claim 1, **characterized in that** the point mutation in the polynucleotide encoding the amino acid sequence of SEQ ID NO: 3 causes aspartic acid at position 372 in the amino acid sequence of SEQ ID NO: 3 to be substituted with a different amino acid; preferably, aspartic acid at position 372 is substituted with asparagine.

3. The bacterium of any one of claims 1-2, **characterized in that** the polynucleotide encoding the amino acid sequence of SEQ ID NO: 3 comprises a nucleotide sequence of SEQ ID NO: 1.

4. The bacterium of any one of claims 1-3, **characterized in that** the polynucleotide sequence having the point mutation is formed from a mutation to the base at position 1114 of a polynucleotide sequence set forth in SEQ ID NO: 1;
preferably, the mutation comprises a mutation of the base at position 1114 of the polynucleotide sequence set forth in SEQ ID NO: 1 from guanine (G) to adenine (A);
preferably, the polynucleotide sequence having the point mutation comprises a polynucleotide sequence set forth in SEQ ID NO: 2.

5. The bacterium of any one of claims 1-4, **characterized in that** the bacterium is a bacterium of the genus *Corynebacterium,* preferably, *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis;* more preferably, *Corynebacterium glutamicum* CGMCC No. 21220 or ATCC 13869.

6. A polynucleotide sequence, **characterized in** comprising a polynucleotide encoding containing an amino acid sequence set forth in SEQ ID NO: 3, wherein aspartic acid at position 372 is substituted with a different amino acid; preferably, aspartic acid at position 372 is substituted with asparagine;
preferably, the polynucleotide sequence comprises a polynucleotide encoding containing an amino acid sequence set forth in SEQ ID NO: 4;
preferably, the polynucleotide sequence is formed from a mutation to the base at position 1114 of a polynucleotide sequence set forth in SEQ ID NO: 1; preferably, the mutation is a mutation of the base at position 1114 of the polynucleotide sequence set forth in SEQ ID NO: 1 from guanine (G) to adenine (A);
preferably, the polynucleotide sequence comprises a polynucleotide sequence set forth in SEQ ID NO: 2.

7. A protein, **characterized in that** the sequence is set forth in SEQ ID NO: 4.

8. A recombinant vector, **characterized in** comprising the polynucleotide sequence of claim 6.

9. A recombinant strain, **characterized in** comprising the polynucleotide sequence of claim 6.

10. A method for producing L-glutamic acid, the method comprising: culturing the bacterium of any of claims 1-5 and recovering L-glutamic acid from the culture.

11. A protein, designated as BBD29 _14900^{D372N} protein, is obtained by a mutation of the amino acid residue at position 372 of BBD29_14900 protein from aspartic acid to asparagine;
the BBD29_14900 protein being (a1) or (a2) or (a3) as follows:
(a1) a protein set forth in SEQ ID NO: 3 in the Sequence Listing;
(a2) a protein derived from a bacterium and having 95% or more identity to (a1) and relating to glutamic acid production by a bacterium;
(a3) a protein derived from (a1) and obtained by subjecting the protein indicated in (a1) to substitution and/or deletion and/or addition of one or several amino acid residues and relating to glutamic acid production by a bacterium.

12. A coding gene of the BBD29 _14900^{D372N} protein of claim 11.

13. An expression cassette or a recombinant vector or a recombinant bacterium having the coding gene of claim 12.

14. Use of the protein of claim 11, the coding gene of claim 12, the expression cassette of claim 13, the recombinant vector of claim 13, or the recombinant bacterium of claim 13;
the use being (I) or (II) as follows:
(I) use in increasing glutamic acid production by a bacterium;
(II) use in the production of glutamic acid.

15. Use of a particular substance;
the use being (I) or (II) as follows:
(I) use in increasing glutamic acid production by a bacterium;
(II) use in the production of glutamic acid;
the particular substance being (d1), (d2), (d3), (cd4), (d5) or (d6) as follows:
(d1) a substance for increasing the expression of BBD29_14900^{G1114A} gene;
(d2) a substance for increasing the abundance of BBD29_14900^{D372N} protein;
(d3) a substance for increasing the activity of BBD29_14900^{D372N} protein;
(d4) a substance for increasing the expression of BBD29_14900 gene;
(d5) a substance for increasing the abundance of BBD29_14900 protein;
(d6) a substance for increasing the activity of BBD29_14900 protein;
the BBD29 _14900^{D372N} protein being the BBD29_14900^{D372N} protein of claim 11;
the BBD29_14900^{G1114A} gene being a gene encoding the BBD29_14900^{D372N} protein;
the BBD29_14900 protein being the BBD29_14900 protein of claim 11;
the BBD29_14900 gene being a gene encoding the BBD29_14900 protein.

16. A recombinant bacterium obtained by overexpressing BBD29_14900^{G1114A} gene or BBD29_14900 gene in a bacterium; the BBD29_14900^{G1114A} gene being the BBD29_14900^{G1114A} gene of claim 15; the BBD29_14900 gene being the BBD29_14900 gene of claim 15.

17. Use of the recombinant bacterium of claim 16 in the preparation of glutamic acid.

18. A method for increasing the production of glutamic acid of a bacterium, comprising the step of:
substituting BBD29_14900 gene in the genome of a bacterium with BBD29_14900^{G1114A} gene; the BBD29_14900^{G1114A} gene being the BBD29_14900^{G1114A} gene of claim 15; the BBD29_14900 gene being the BBD29_14900 gene of claim 15.

19. A method for increasing the production of glutamic acid of a bacterium, comprising the step of: overexpressing BBD29_14900^{G1114A} gene in a bacterium or overexpressing BBD29_14900 gene in a bacterium, or increasing the abundance of BBD29 _14900^{D372N} protein in a bacterium or increasing the abundance of BBD29_14900 protein in a bacterium, or increasing the activity of BBD29 _14900^{D372N} protein in a bacterium or increasing the activity of BBD29_14900 protein in a bacterium;
the BBD29 _14900^{D372N} protein being the BBD29_14900^{D372N} protein of claim 11;
the BBD29_14900^{G1114A} gene being a gene encoding the BBD29_14900^{D372N} protein;
the BBD29_14900 protein being the BBD29_14900 protein of claim 11;
the BBD29_14900 gene being a gene encoding the BBD29_14900 protein.

20. Use of BBD29 _14900^{D372N} protein or BBD29_14900 protein in the regulation of the production of glutamic acid of a bacterium; the BBD29 _14900^{D372N} protein being the BBD29_14900^{D372N} protein of claim 11; the BBD29_14900 protein being the BBD29_14900 protein of claim 11.
